# EUROPEAN PATENT APPLICATION

(11) **EP 1 662 006 A1**
(43) Date of publication of application: **31.05.2006**
(21) Application number: 04771076.9
(22) Date of filing: 30.07.2004
(51) Int. Cl.: C12Q 1/68, C12N 15/09

(54) **PARTIALLY DOUBLE STRANDED NUCLEIC ACID MOLECULE**

(30) Priority: 30.07.2003 JP 2003282311
(71) Applicant: The Circle for the Promotion of Science and Engineering, Tokyo 152-8550 (JP)
(72) Inventor: MARUYAMA, Atsushi, TOKYO INSTITUTE OF TECHNOLOGY, Yokohama-shi, Kanagawa 2268502 (JP); SATO, Yuichi, TOKYO INSTITUTE OF TECHNOLOGY, Yokohama-shi, Kanagawa 2268502 (JP); HIRATA, Kazuya, TOKYO INSTITUTE OF TECHNOLOGY, Yokohama-shi, Kanagawa 2268502 (JP)
(74) Representative: Woods, Geoffrey Corlett
(86) International application number: PCT/JP2004/010916
(87) International publication number: WO 2005/012571

(57) **Abstract**

A nucleic acid molecule for detecting a nucleic acid molecule is provided which is a partially double-stranded nucleic acid molecule consisting of (a) a single-stranded nucleic acid molecule complementary to the nucleic acid molecule to be detected and (b) one or two single-stranded nucleic acid molecules that hybridize with part of the single-stranded nucleic acid molecule (a), wherein the region of the partially double-stranded nucleic acid molecule which assumes a single-stranded structure is complementary to a region comprising the recognition site in the nucleic acid molecule to be detected. Slight differences in nucleotide sequences can be easily recognized by using the nucleic acid molecule as a probe.

SEQUENCE LISTING

<110> The Circle for the Promotion of Science and Engineering

<120> PARTIAL DOUBLE-STRANDED NUCLEIC ACID MOLECULAR

<130> N97208

<150> EP 04771076.9
<151> 2004-07-30

<140> PCT/JP2004/010916
<141> 2004-07-30

<140> JP 2008-282311
<141> 2003-07-30

<160> 18

<170> PatentIn Ver. 2.1

<210> 1
<211> 14
<212> DNA
<213> Artificial Sequence

<220>
<223> Description of Artificial Sequence: probe

<400> 1
gaaataatca atga                                                    14


<210> 2
<211> 19
<212> DNA
<213> Artificial Sequence

<220>
<223> Description of Artificial Sequence: probe

<400> 2
tcattgatta tttcccagg                                               19


<210> 3
<211> 19
<212> DNA
<213> Artificial Sequence

<220>
<223> Description of Artificial Sequence: probe

<400> 3
cctgggaaat aatcaatga                                               19


<210> 4
<211> 19
<212> DNA

<213> Artificial Sequence

<220>
<223> Description of Artificial Sequence: probe

<400> 4
cccgggaaat aatcaatga                                                    19


<210> 5
<211> 19
<212> DNA
<213> Artificial Sequence

<220>
<223> Description of Artificial Sequence: probe

<400> 5
cctgggaaat actcaatga                                                    19


<210> 6
<211> 19
<212> DNA
<213> Artificial Sequence

<220>
<223> Description of Artificial Sequence: probe

<400> 6
cctgggaaat aatcaacga                                                    19


<210> 7
<211> 14
<212> DNA
<213> Artificial Sequence

<220>
<223> Description of Artificial Sequence: probe

<400> 7
gaaataatca atga                                                         14


<210> 8
<211> 19
<212> DNA
<213> Artificial Sequence

<220>
<223> Description of Artificial Sequence: probe

<400> 8
tcattgatta tttcccggg                                                    19


<210> 9
<211> 20
<212> DNA
<213> Artificial Sequence

<220>
<223> Description of Artificial Sequence: probe

<400> 9
aaaagtgaaa aagataagaa                                                    20


<210> 10
<211> 25
<212> DNA
<213> Artificial Sequence

<220>
<223> Description of Artificial Sequence: probe

<400> 10
ttcttatctt tttcactttt atcgt                                              25


<210> 11
<211> 25
<212> DNA
<213> Artificial Sequence

<220>
<223> Description of Artificial Sequence: probe

<400> 11
acgataaaag tgaaaaagat aagaa                                              25


<210> 12
<211> 25
<212> DNA
<213> Artificial Sequence

<220>
<223> Description of Artificial Sequence: probe

<400> 12
acaataaaag tgaaaaagat aagaa                                              25


<210> 13
<211> 25
<212> DNA
<213> Artificial Sequence

<220>
<223> Description of Artificial Sequence: probe

<400> 13
acgataaaag tgaaaaaaat aagaa                                              25


<210> 14
<211> 25
<212> DNA
<213> Artificial Sequence

<220>
<223> Description of Artificial Sequence: probe

<400> 14
ttcttatctt tttcactttt attgt                                          25


<210> 15
<211> 40
<212> DNA
<213> Artificial Sequence

<220>
<223> Description of Artificial Sequence: probe

<400> 15
aaaagtgaaa aagataagaa ggaaaagaaa ccaactgtca                          40


<210> 16
<211> 45
<212> DNA
<213> Artificial Sequence

<220>
<223> Description of Artificial Sequence: probe

<400> 16
tgacagttgg tttcttttcc ttcttatctt tttcactttt atcgt                    45


<210> 17
<211> 45
<212> DNA
<213> Artificial Sequence

<220>
<223> Description of Artificial Sequence: probe

<400> 17
acgataaaag tgaaaaagat aagaaggaaa agaaaccaac tgtca                    45

<210> 18
<211> 45
<212> DNA
<213> Artificial Sequence

<220>
<223> Description of Artificial Sequence: probe

<400> 18
acaataaaag tgaaaaagat aagaaggaaa agaaaccaac tgtca                    45

## Description

### TECHNICAL FIELD

The present invention relates to a nucleic acid molecule that can be used as a probe or primer, and to a use therefor. Using this nucleic acid molecule as a probe allows single-nucleotide mutations and other slight differences in gene sequences to be recognized rapidly and easily.

### BACKGROUND ART

The only conventional techniques with sufficient ability to recognize single-nucleotide mutations and other slight differences in nucleotide sequences are those using enzyme reactions. Analytic methods using enzymes are often complicated and problematic from the standpoint of operability, cost, analysis time and the like.

One conventional technique that does not use enzymes is the molecular beacon method, but this requires very careful design of probes for analyzing single-nucleotide mutations, posing problems for purposes of high throughput. Problems of diagnostic accuracy may also occur in the molecular beacon method because the probe nucleic acids must include nucleotide sequences not involved in recognizing differences.

There have also been recent publications relating to partially double-stranded probes having both single-stranded and double-stranded parts (International Patent Publication 02/50308, pamphlet, Patent Publication 2004-511227 (International Patent Publication 02/30946, pamphlet), Qingge Li, Guoyan Luan, Qiuping Guo and Jixuan Liang, *Nucleic Acids Research* 2002, Vol. 30, No. 2). These publications describe the recognition of single-nucleotide mutations and the like using probes with partially double-stranded structures, but do not say what location on the probe should be designed to correspond to the single-nucleotide mutation.

### DISCLOSURE OF THE INVENTION

It is an object of the present invention to provide a probe capable of easily recognizing slight differences in nucleotide sequences.

As a result of exhaustive research aimed at solving the aforementioned problems, the inventors discovered that a target nucleic acid molecule alone could be detected with high sensitivity by designing a probe using as the probe structure a partially double-stranded structure having both a single-stranded part and a double-stranded part, wherein the single-stranded part corresponds to the anticipated position of a gene polymorphism and the like in a nucleic acid molecule to be detected, and perfected the present invention based on this finding.

That is, the present invention provides (1) through (16) below.
(1) A nucleic acid molecule for detecting a nucleic acid molecule, which is a partially double-stranded nucleic acid molecule comprising (a) a single-stranded nucleic acid molecule complementary to the nucleic acid molecule to be detected and (b) one or two single-stranded nucleic acid molecules which hybridize with part of the single-stranded nucleic acid molecule (a), wherein the region of the partially double-stranded nucleic acid molecule which assumes a single-stranded structure is complementary to a region comprising a recognition site on the nucleic acid molecule to be detected.
(2) A nucleic acid molecule according to (1), wherein the length of the region that assumes a double-stranded structure is 10 to 200 nucleotides.
(3) A nucleic acid molecule according to (1) or (2), wherein the length of the region that assumes a single-stranded structure is 1 to 10 nucleotides.
(4) A nucleic acid molecule according to any of (1) through (3), wherein either one of the single-stranded nucleic acid molecule (a) or the single strand nucleic-acid molecule (b) is labeled with a donor fluorescent dye, while the other is labeled with an acceptor fluorescent dye.
(5) A nucleic acid molecule according to any one of (1) through (4), wherein the single-stranded nucleic acid molecule (a) and the single-stranded nucleic acid molecule (b) are connected by means of a linker.
(6) A nucleic acid molecule according to (5), wherein the linker is a nucleic acid.
(7) A nucleic acid chip comprising a nucleic acid molecule according to any of (1) through (6) above fixed on a substrate.
(8) A method for detecting a nucleic acid molecule, comprising a step in which a nucleic acid according to any of (1) through (6) above is brought into contact with a nucleic acid molecule to be detected under conditions that allow hybridization.
(9) A method for detecting a nucleic acid molecule according to (8), wherein a nucleic acid according to any of (1) through (6) above is brought into contact with a nucleic acid molecule to be detected in the presence of an amine or quaternary ammonium salt.
(10) A method for detecting a nucleic acid molecule according to (8) or (9), wherein a nucleic acid according to any of (1) through (6) above is brought into contact with a nucleic acid molecule to be detected in the presence of a cationic polymer.
(11) A mismatched sequence detection method for detecting a sequence mismatch between a sample single-stranded nucleic acid molecule and a standard single-stranded nucleic acid molecule, comprising a step in which a nucleic acid molecule which is a partially double-stranded nucleic acid molecule comprising (a) a single-stranded nucleic acid molecule complementary to the sample single-stranded nucleic acid molecule and (b) one or two single-stranded nucleic acid molecules which hybridize with part of the single-stranded nucleic acid molecule (a), the region which assumes a single-stranded structure in the partially double-stranded nucleic acid molecule being complementary to a region comprising a site where the sequence mismatch is anticipated in the sample single-stranded nucleic acid molecule, is brought into contact with the sample single-stranded nucleic acid molecule under conditions which allow hybridization.
(12) A mismatched sequence detection method according to (11), wherein the partially double-stranded nucleic acid molecule is brought into contact with the sample nucleic acid molecule in the presence of an amine or quaternary ammonium salt.
(13) A mismatched sequence detection method according to (11) or (12), wherein the partially double-stranded nucleic acid molecule is brought into contact with the sample nucleic acid molecule in the presence of a cationic polymer.
(14) A nucleic acid molecule detection method wherein a first detection probe complementary to a region which does not comprise a recognition site in a nucleic acid molecule to be detected is added to a sample comprising the nucleic acid molecule to be detected, and a second detection probe which comprises a nucleotide sequence identical to that of the first detection probe and which is complementary to the region comprising a recognition site in the nucleic acid molecule to be detected is then added, the nucleic acid molecule to be detected, the first detection probe and the second probe are brought together under conditions that allow hybridization, and nucleic acid molecule detection is then performed using as the marker either binding between the nucleic acid molecule to be detected and the second detection probe, or dissociation between the nucleic acid molecule to be detected and the first detection probe.
(15) A nucleic acid molecule detection method according to (14) wherein the nucleic acid molecule to be detected, the first detection probe and the second probe are brought together in the presence of an amine or quaternary ammonium salt.
(16) A nucleic acid molecule detection method according to (14) or (15), wherein the nucleic acid molecule to be detected, the first detection probe and the second probe are brought together in the presence of a cationic polymer.

Using the nucleic acid molecule of the present invention as a probe or the like allows the recognition of single-nucleotide mutations and other slight differences in nucleotide sequences. Moreover, using the nucleic acid molecule of the present invention as a probe or the like it is possible to detect nucleic acid molecules in a simple operation without the need to strictly control temperature and the like.

### BRIEF DESCRIPTION OF THE DRAWINGS

Figure 1 shows the structure of the nucleic acid molecule of the present invention.
Figure 2 shows the process of strand exchange between the nucleic acid molecule of the present invention and a target nucleic acid molecule.
Figure 3 is an outline of a detection method that does not use the nucleic acid molecule of the present invention.
Figure 4 shows complementarity and other relationships among the cytochrome P450 gene-related oligodeoxynucleotides (ODN) used in the examples.
Figure 5 shows complementarity and other relationships among the ABC transporter gene-related ODNs (20-25mers) used in the examples.
Figure 6 shows complementarity and other relationships among the ABC transporter gene-related ODNs (40-45mers) used in the examples.
Figure 7 shows changes over time in fluorescent strength for full-matched and mismatched ODNs (fluorescent dye: FITC).
Figure 8 shows changes over time in fluorescent strength for full-matched and mismatched ODNs (fluorescent dye: FITC) at high salt concentrations.
Figure 9 shows changes over time in fluorescent strength for full-matched and mismatched ODNs (fluorescent dye: TAMRA).
Figure 10 shows changes over time in strand exchange rates for full-matched and mismatched (mismatch corresponding to single-stranded part of probe) ODNs.
Figure 11 shows changes over time in strand exchange rates for full-matched and mismatched (mismatch corresponding to double-stranded part of probe) ODNs.
Figure 12 shows relative values for the strand-exchange rate constants of full-matched and mismatched ODNs at various temperatures.
Figure 13 shows changes over time in strand-exchange rates for full-matched and mismatched ODNs in the absence of tetramethylammonium chloride.
Figure 14 shows changes over time in strand-exchange rates for full-matched and mismatched ODNs in the presence of tetramethylammonium chloride.
Figure 15 shows the results of the strand-exchange test (using cyctochrome P450 gene-related ODNs) of Example 9.
Figure 16 shows the results of the strand-exchange test (using ABC transporter gene-related ODNs) of Example 9.
Figure 17 shows changes over time in strand-exchange rates for full-matched and mismatched ODNs (45mers).

### BEST MODE FOR CARRYING OUT THE INVENTION

The present invention is explained in detail below.

The nucleic acid molecule of the present invention is a nucleic acid molecule for detecting nucleic acid molecules, and is a partially double-stranded nucleic acid molecule comprising (a) a single-stranded nucleic acid molecule complementary to a nucleic acid molecule to be detected and (b) one or two single-stranded nucleic acid molecules which hybridize with one part of single-stranded nucleic acid molecule (a), wherein the region of the partially double-stranded nucleic acid molecule which assumes a single-stranded structure is complementary to a region comprising a recognition site in the nucleic acid molecule to be detected.

In the present invention, the phrase "nucleic acid molecule" normally means DNA, but also includes RNA and nucleic acid-like molecules (such as peptide nucleic acids, LNA and morpholinonucleotides) and the like.

A "recognition site" is the anticipated site of a difference in sequence between the nucleic acid molecule to be detected and a similar nucleic acid molecule. The anticipated site of a genetic polymorphism may normally be a recognition site. A feature of the nucleic acid molecule of the present invention is that this recognition site corresponds to a single-stranded structure part. As discussed above, although partially double-stranded nucleic acid molecules are already known, in all of these known nucleic acid molecules the recognition site corresponds to a double-stranded structure part (see for example Patent Publication 2004-511227 (International Patent Publication 02/30946), pamphlet, example 1).

There are no particular limits on the nucleic acid molecule to be detected, but a gene comprising a single-nucleotide mutation or the like is desirable as the object of detection because the nucleic acid molecule of the present invention is effective for recognizing single-nucleotide mutations.

There may be either one or two single-stranded nucleic acid molecules (b) . If there is one, one side assumes a double-stranded structure while the other assumes a single-stranded structure, while if there are two, both sides assume a double-stranded structure with a single strand in the central part.

There are no particular limits on the length of the region that assumes a double-stranded structure in the nucleic acid molecule of the present invention, but a normal length is 10 to 200 nucleotides. The best length depends on the intended use of the nucleic acid molecule, and for example about 10 to 60 nucleotides is desirable for purposes of hybridization in solution, while 15 to 100 nucleotides is desirable for purposes of fixing on a nucleic acid chip or the like.

There are no particular limits on the length of the region that assumes a single-stranded structure in the nucleic acid molecule of the present invention, but normally it is about 1 to 10 nucleotides or preferably 1 to 7 nucleotides

Single-stranded nucleic acid molecule (a) and single-stranded nucleic acid molecule (b) may be connected by means of a linker. The linker may be a nucleic acid or another substance.

There are no particular limits on the method of labeling the nucleic acid molecule of the present invention, and one example is a method of labeling either single-stranded nucleic acid molecule (a) or single-stranded nucleic acid molecule (b) with a donor fluorescent dye (for example, fluorescein isothiocyanate or the like) and labeling the other with an acceptor fluorescent dye (for example, tetramethylrhodamine or the like). In this labeling method, dissociation between single-stranded nucleic acid molecule (a) and single-stranded nucleic acid molecule (b) (signifying hybridization between single-stranded nucleic acid molecule (a) and the nucleic acid molecule to be detected) results in fluorescence which can be used as the marker for detecting the nucleic acid molecule to be detected.

When using the nucleic acid molecule of the present invention as a probe for DNA detection or the like, only 1 type of probe may be used or 2 or more can be used simultaneously. An example of a case when 2 or more types of probes are used is when determining the genotype of a living organism as described below.

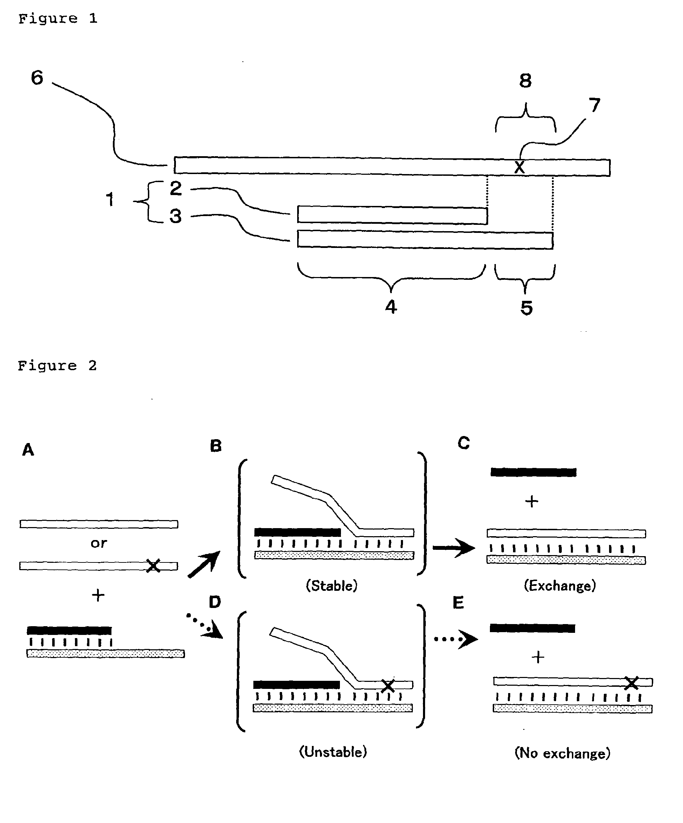

The structure of the nucleic acid molecule of the present invention is explained using Figure 1. Nucleic acid molecule 1 of the present invention is composed of short single strand 2 and long single strand 3, and has double-stranded part 4 and single-stranded part 5 due to the difference in length between the two single strands. Long single strand 3 is complementary to nucleic acid molecule 6 to be detected, and single-stranded part 5 of long single strand 3 is complementary to region 8 comprising recognition site 7 in nucleic acid molecule 6 to be detected.

The principles by which the nucleic acid molecule of the present invention detects DNA and the like with high sensitivity are explained using Figure 2.

When the nucleic acid molecule of the present invention and a target nucleic acid molecule are brought together (Figure 2A), the short strand of the nucleic acid molecule of the present invention is replaced by the target nucleic acid molecule, and the long strand of the nucleic acid molecule of the present invention forms a double-stranded structure with the target nucleic acid molecule (Figure 2C). As shown in Figure 2B, this strand exchange involves an intermediate transitional state. Homology between the single-stranded part and the target nucleic acid promotes the formation of the transitional complex.

However, when the target nucleic acid molecule includes a mismatched sequence, formation of a transitional complex as mentioned above is not promoted (Figure 2D), and consequently there is no strand exchange (Figure 2E). Slight differences in sequence can be recognized on the basis of ease of formation of such transitional complexes.

A nucleic acid chip can be prepared by fixing the nucleic acid molecule of the present invention on a substrate. The substrate used may be one normally used for DNA chips. Similarly, the fixing method may be one used for ordinary DNA chips. Either single-stranded nucleic acid molecule (a) or single-stranded nucleic acid molecule (b) may be fixed on the substrate.

Detection of nucleic acid molecules with the nucleic acid molecule of the present invention can be accomplished for example by bringing the nucleic acid molecule of the present invention into contact with the nucleic acid molecule to be detected under conditions that allow hybridization. The conditions for bringing the two nucleic acid molecules into contact are not particularly limited as long as they are conditions that allow hybridization, but preferably the two are brought into contact in the presence of an amine (such as a primary amine, secondary amine, tertiary amine or the like) or quaternary ammonium salt.

Examples of the amines and quaternary ammonium salts used here include isopropylamine, butylamine, pentylamine, hexylamine, dimethylamine, trimethylamine, diethylamine, triethylamine, diisopropylamine, tetramethylammonium chloride, tetramethylammonium bromide, tetraethylammonium chloride, tetraethylammonium bromide, tetrapropylammonium chloride, tetrapropylammonium bromide, tetrabutylammonium chloride, tetrabutylammonium bromide and the like, and of these tetramethylammonium chloride is used by preference. There is no particular limit on the amount of the amine or quaternary ammonium salt used, but normally it can be added to a concentration of about 0.5 to 4 M.

The nucleic acid molecules are preferably brought into contact in the presence of a cationic polymer.

Examples of cationic polymers to be used include the hydrophilic graft copolymer disclosed in International Patent Publication 03/018841 (pamphlet) (a graft copolymer having as the main chain a polymer composed of monomers capable of forming cationic bases, with a hydrophilic polymer as the side chain) and a PC polymer (a polymer formed by polymerization of a monomer containing a phosphorylcholine-like group and a cationic monomer having a cationic group). Specific compounds include all compounds disclosed in International Patent Publication 03/018841 (pamphlet), and typical examples are such hydrophilic graft copolymers as dextran side-chain modified α-poly(L-lysine), dextran side-chain modified ε-poly(L-lysine) and dextran side-chain modified polyallylamine, and such PC polymers as a polymer formed by polymerization of 2-(methacryloyloxy)ethyl-2'-(trimethylammonio)ethyl phosphate with an aminoethyl acrylate (hydrochloride) having a primary amino group and polymers formed by polymerization of 2-(methacryloyloxy)ethyl-2'-(trimethylammonio)ethyl phosphate with [2-(acryloyloxyamino)ethyl]trimethylammonium chloride having a quaternary ammonium group and 2-(methacryloyloxy)ethyl-2'-(trimethylammonio)ethyl phosphate with 2-hydroxy-3-methacryloyloxypropyl trimethylammonium chloride. There are no particular limits on the amount of cationic polymer used, but the ratio of cationic groups of the cationic polymer to phosphoric acid groups in the nucleic acid molecule is preferably 0.01 to 1000.

The nucleic acid molecule of the present invention can be used for detecting mismatched sequences. That is, in the detection of a sequence mismatch between a sample single-stranded nucleic acid molecule and a standard single-stranded nucleic acid molecule, the mismatched sequence can be detected by bringing the nucleic acid molecule of the present invention into contact with the sample single-stranded nucleic acid molecule under conditions that allow hybridization. In this case, the anticipated site of the mismatched sequence corresponds to the region that assumes a single-stranded structure in the nucleic acid molecule of the present invention.

The nucleic acid molecule of the present invention can be used for determining the genotype of a living organism or in other words for determining whether the genotype of a given organism is a wild type, heterozygous mutant or homozygous mutant. Specifically, a nucleic acid molecule for detecting the wild type gene and a nucleic acid molecule for detecting the mutant type gene are prepared and each labeled in a different way (such as with difference fluorescent dyes), and the two different nucleic acid molecules are brought together with a nucleic acid molecule derived from the test organism. If the wild type gene alone is detected the test organism is of the wild type, if the mutant gene alone is detected the test organism is a homozygous mutant, and if both genes are detected the test organism is a heterozygous mutant.

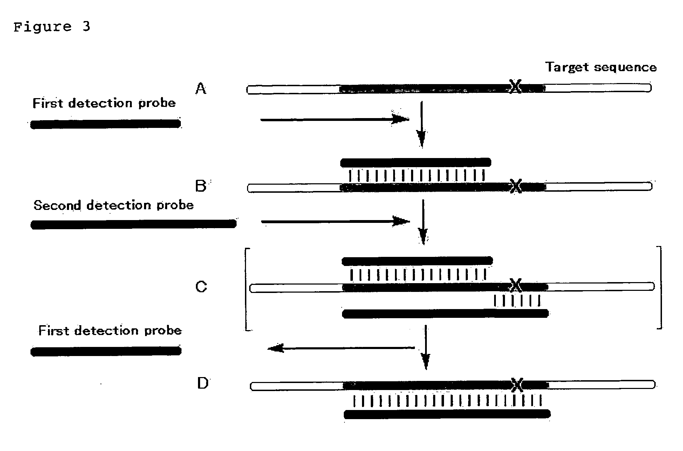

A nucleic acid molecule can also be detected using the principles explained in Figure 3 without using the nucleic acid molecule of the present invention. For example, a method such as the following is conceivable.

A first detection probe complementary to a region not comprising the recognition site in the nucleic acid molecule to be detected is added to a sample comprising the nucleic acid molecule to be detected. The first detection probe thus hybridizes with the nucleic acid molecule to be detected (Figure 3B) . The recognition site of the nucleic acid molecule to be detected remains in a single-stranded state.

Next, a second detection probe is added which comprises the same nucleotide sequence as the first detection probe and which is complementary to a region comprising the recognition site in the nucleic acid molecule to be detected. In this case, if the sequence of the recognition site of the nucleic acid molecule to be detected is complementary to the sequence of the corresponding site on the second detection probe, a transitional complex is formed as shown in Figure 3C, and the first detection probe is dissociated (Figure 3D). Conversely, if the sequence of the recognition site of the nucleic acid molecule to be detected is not complementary to the sequence of the corresponding site on the second detection probe, no transitional complex forms, and the first detection probe is not dissociated.

Thus, using as the marker either binding between the nucleic acid molecule to be detected and the second detection probe or dissociation between the nucleic acid molecule to be detected and the first detection probe it is possible to determine with great precision whether or not the nucleic acid to be detected is present in a sample.

### [Examples]

The present invention is explained in more detail below using example.

The ODNs used in the examples are explained first.

### (A) Cytochrome P450 gene-related ODNs

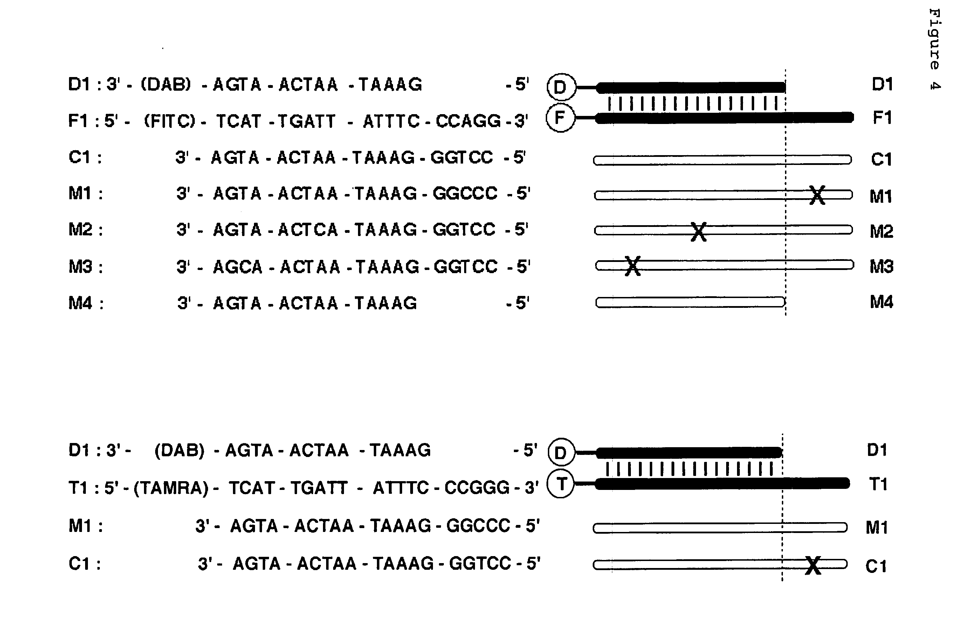

8 ODNs (F1, D1, C1, M1, M2, M3, M4 and T1) were used as ODNs related to the cytochrome P450 gene. Figure 4 shows the relationships among these ODNs.

### (1) F1 and D1

F1 is an ODN labeled on the 5' end with fluorescein isothiocyanate (FITC), while D1 is an ODN labeled on the 3' end with dabsyl (DAB). F1 and D1 are complementary to one another, but are 19 nucleotides and 14 nucleotides in length, respectively, so that even if F1 forms double-stranded DNA with D1 a continuous single-stranded part consisting of 5 nucleotides remains at the 3' end.

The nucleotide sequences of F1 and D1 are represented by SEQ ID NOS 2 and 1, respectively.

### (2) C1

C1 is a fully complementary unlabeled ODN that forms a complementary double strand with F1.

The nucleotide sequence of C1 is represented by SEQ ID NO 3. (3) M1, M2 and M3 have single-nucleotide mutations near the 5' end, in the center and near the 3' end, respectively, resulting in mismatched nucleotide pairs when they form double strands with F1. Each of these ODNs is 19 nucleotides in length.

The nucleotide sequences of M1, M2 and M3 are represented by SEQ ID NOS 4, 5 and 6 respectively.

### (4) M4

M4 has the same sequence as D1 and a length of 14 nucleotides.

The nucleotide sequence of M4 is represented by SEQ ID NO 7. (5) T1

T1 is an ODN labeled at the 5' end with tetramethylrhodamine (TAMRA). T1 is complementary to D1 but is 19 nucleotides in length, so that as in the case of F1, even if T1 forms double-stranded DNA with D1 a continuous single-stranded part consisting of 5 nucleotides remains at the 3' end.

In contrast with F1, T1 is fully complementary to M1 but forms a mismatched nucleotide pair with C1 near the 5' end.

The nucleotide sequence of T1 is represented by SEQ ID NO 8.

### (B) ABC transporter gene-related ODNs.

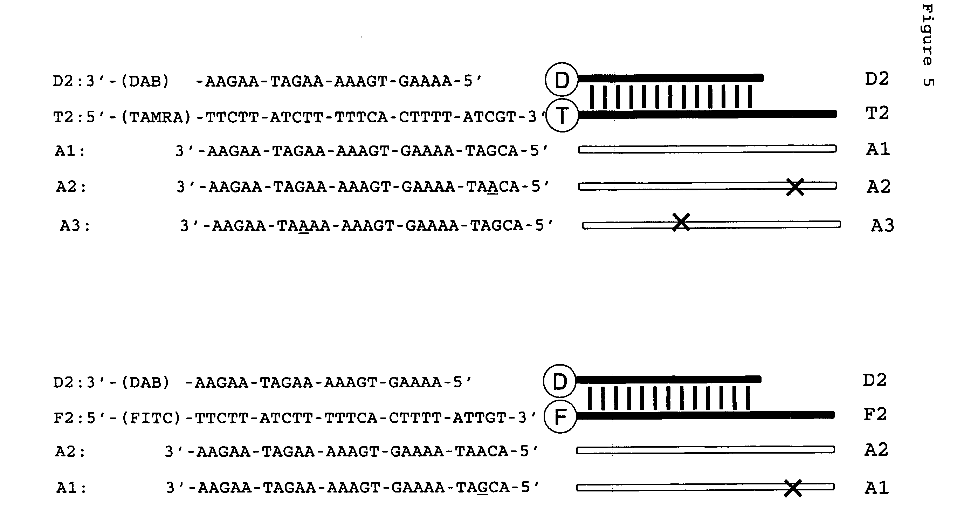

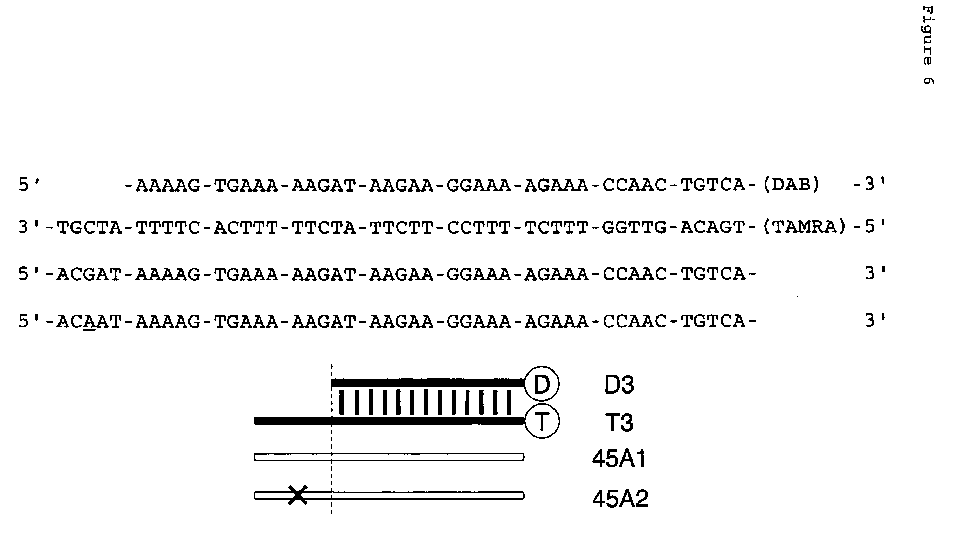

10 different ODNs, F2, D2, A1, A2, A3, T2, D3, T3, 45A1 and 45A2, were used as ABC transporter gene-related ODNs. The relationships among F2, D2, A1, A2, A3 and T2 and the relationships among D3, T3, 45A1 and 45A2 are shown in Figures 5 and 6, respectively.
(1) F2 and D2
   F2 is an ODN labeled at the 5' end with FITC, while D2 is labeled at the 3' end with DAB. F2 and D2 are complementary to one another, but are 25 nucleotides and 20 nucleotides in length, respectively, so that even if F2 forms double-stranded DNA with D2 a continuous single-stranded part consisting of 5 nucleotides remains at the 3' end. The single-stranded part of F2/D2 is an AT-rich sequence.
   The nucleotide sequences of F2 and D2 are represented by SEQ ID NO 14 and SEQ ID NO 9, respectively.
(2) A1
   A1 is a fully complementary unlabeled ODN capable of forming a complementary double strand with T2.
   The nucleotide sequence of A1 is represented by SEQ ID NO 11.
(3) A2 is an ODN 25 nucleotides in length having a single-nucleotide mutation near the 5' end which forms a mismatched nucleotide pair in a double strand with T2.
   The nucleotide sequence of A2 is represented by SEQ ID NO 12.
(4) A3
   A3 is an ODN 25 nucleotides in length having a single-nucleotide mutation in the center which forms a mismatched nucleotide pair in a double strand with T2.
   The nucleotide sequence of A3 is represented by SEQ ID NO 13.
(5) T2
   T2 is an ODN labeled at the 5' end with TAMRA. T2 is complementary to D2 but is 25 nucleotides in length, so that as in the case of F2, even if it forms double-stranded DNA with D2 a continuous single-stranded part consisting of 5 nucleotides remains at the 3' end.
   The nucleotide sequence of T2 is represented by SEQ ID NO 10.
(6) T3 and D3
   T3 is an ODN labeled at the 5' end with TAMRA, while D3 is labeled at the 3' end with DAB. Although T3 and D3 have complementary nucleotide sequences they are 45 nucleotides and 40 nucleotides in length, respectively, so that even if T3 forms double-stranded DNA with D3 a continuous single-stranded part of 5 nucleotides remains at the 3' end.
   The nucleotide sequences of T3 and D3 are represented by SEQ ID NO 16 and SEQ ID NO 15, respectively.
(7) 45A1
   45A1 is a fully complementary unlabeled ODN capable of forming a complementary double strand with T3.
   The nucleotide sequence of 45A1 is represented by SEQ ID NO 17.
(8) 45A2
   45A2 is an ODN 45 nucleotides in length having a single-nucleotide mutation at the 5' end which forms a mismatched nucleotide pair in a double strand with T3.
   The nucleotide sequence of 45A2 is represented by SEQ ID NO 18.
   The aforementioned ODNs were purchased from FASMAC Co., Ltd. and purified by reverse phase chromatography.

### [Example 1]

Equal molar amounts of F1 and D1 were added to PBS buffer ([Na⁺] = 150 mM, pH 7.2). This buffer was heated to 90°C and then gradually cooled to prepare a solution containing 12 nM of partially double-stranded F1/D1 probe. This solution was kept at 20°C, and C1, M1, M2, M3 and M4 were added as single strands to 60 nM to initiate strand-exchange reactions. The progress of strand exchange was detected from the recovery of FITC fluorescence (excitation wavelength 490 nm, fluorescence wavelength 520 nm) extinguished by DAB.

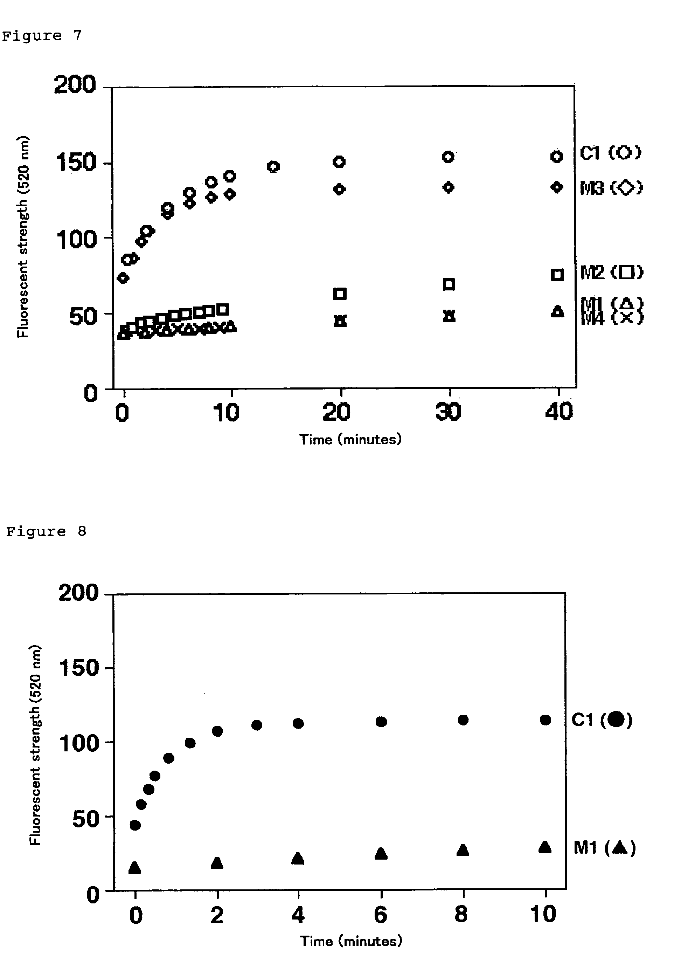

Changes over time in fluorescent strength after the start of the reaction are shown in Figure 7. Fluorescent strength 5 minutes, 10 minutes and 20 minutes after the start of the reaction are shown in Table 1. Additionally, the relative strand-exchange rate constants for each single strand are shown in Table 2.

**[Table 1]**

| Sequence | 5 minutes | 10 minutes | 20 minutes |
|---|---|---|---|
| C1 | 123.7 | 140.9 | 150.1 |
| M1 | 38.6 | 40.8 | 44.1 |
| M2 | 47.8 | 53.2 | 61.8 |
| M3 | 119.3 | 128.7 | 132.0 |
| M4 | 38.7 | 40.5 | 44.1 |

**[Table 2]**

| Sequence | Relative strand exchange rate constant |
|---|---|
| C1 | 1.00 |
| M1 | 0.02 |
| M2 | 0.06 |
| M3 | 1.06 |
| M4 | 0.02 |

As shown in the figure and tables, the fully complementary single strand (C1) underwent strand exchange more rapidly that the single strands with mutations (M1, M2, M3). The rate difference was greater between C1 and M1, which had a mutation at a site near the 5' end (corresponding to the single-stranded part of F1) than between C1 and M2, which had a mutation in the central part, and there was almost no difference between C1 and M3, which had a mutation at a site near the 3' end.

From this it appears that a mismatched sequence can be detected with high sensitivity by designing a partially double-stranded probe so as to correspond to the single-stranded part of the mismatched sequence.

### [Example 2]

Strand-exchange reactions were performed as in Example 1 except that the PBS buffer was given a sodium ion concentration of 1000 mM, and two single strands (C1 and M1) were added.

Changes over time in fluorescent strength after the start of the reaction are shown in Figure 8. The relative strand-exchange rate constants of C1 and M1 are also shown in Table 3.

**[Table 3]**

| Salt concentration - | Sequence | Relative strand exchange rate constant |
|---|---|---|
| 150 mM | C1 | 1.00 |
| 150 mM | M1 | 0.02 |
| 1000 mM | C1 | 5.36 |
| 1000 mM | M1 | 0.08 |

As shown in the table, as in Example 1 there was a large difference in strand-exchange rates between the single strand with the mutation (M1) and the fully complementary single strand (C1) even at a sodium ion concentration of 1000 mM. Raising the sodium ion concentration also raised the strand-exchange rate for both the single strand with the mutation and the fully complementary single strand.

### [Example 3]

Strand-exchange reactions were performed as in Example 1 except that T1/D1 was used as the partially double-stranded probe, the two single strands C1 and M1 were added, and the progress of strand exchange was detected from the recovery of TAMRA fluorescence (excitation wavelength 540 nm, fluorescence wavelength 570 nm).

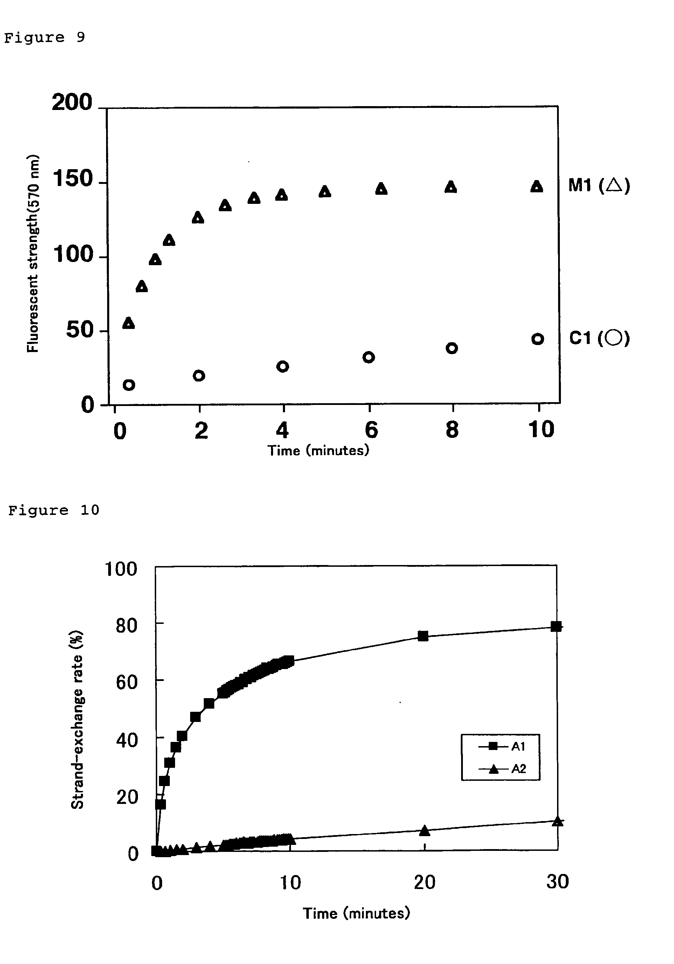

Changes over time in fluorescent strength after the start of the reaction are shown in Figure 9. Fluorescent strength 5 minutes, 10 minutes and 20 minutes after the start of the reaction is also shown in Table 4. The relative strand-exchange rate constants for each single strand are shown in Table 5.

**[Table 4]**

| Sequence | 2 minutes | 5 minutes | 10 minutes |
|---|---|---|---|
| C1 | 19.2 | 28.4 | 42.8 |
| M1 | 126.1 | 143.4 | 146.1 |

**[Table 5]**

| Sequence | Relative strand-exchange rate constant |
|---|---|
| M1 | 1.00 |
| C1 | 0.02 |

As shown in the figure and tables, as in Example 1 there was a large difference in strand exchange speeds between the single strand with the mutation (C1) and the fully complementary single strand (M1). It appears from these results that a partially double-stranded probe can detect not only differences between A-T and A-C nucleotide pairs but also differences between G-C and G-T nucleotide pairs, which are more difficult to detect.

### [Example 4]

Equal molar amounts of T2 and D2 were added to PBS buffer ([Na⁺] = 150 mM, pH 7.2). This buffer was heated to 90°C and then gradually cooled to prepare a solution containing 12 nM of partially double-stranded T2/D2 probe. This solution was kept at 20°C, and A1 and A2 were added as single strands to concentrations of 60 nM to initiate strand-exchange reactions. The progress of strand exchange was detected from the recovery of TAMRA fluorescence extinguished by DAB.

Changes over time in strand exchange rates after the start of the reaction (given a value of 100% for fluorescent strength after annealing performed after following the process of strand exchange) are shown in Figure 10. Fluorescent strength 5 minutes, 10 minutes and 20 minutes after the start of the reaction is also shown in Table 6. The relative strand-exchange rate constants for each single strand are shown in Table 7.

**[Table 6]**

| Sequence | 5 minutes | 10 minutes | 20 minutes |
|---|---|---|---|
| A1 | 82.2 | 95.9 | 106.6 |
| A2 | 12.8 | 14.8 | 17.7 |

**[Table 7]**

| Sequence | Relative strand-exchange rate constant |
|---|---|
| A1 | 1.00 |
| A2 | 0.02 |

As shown in the figure and tables, there was a large difference in strand-exchange rates between the single strand having a mutation corresponding to the single-stranded part of T2 (A2) and the fully complementary single strand (A1).

### [Comparative Example 1]

Strand-exchange reactions were performed as in Example 4 except that the single strands added were A1 and A3.

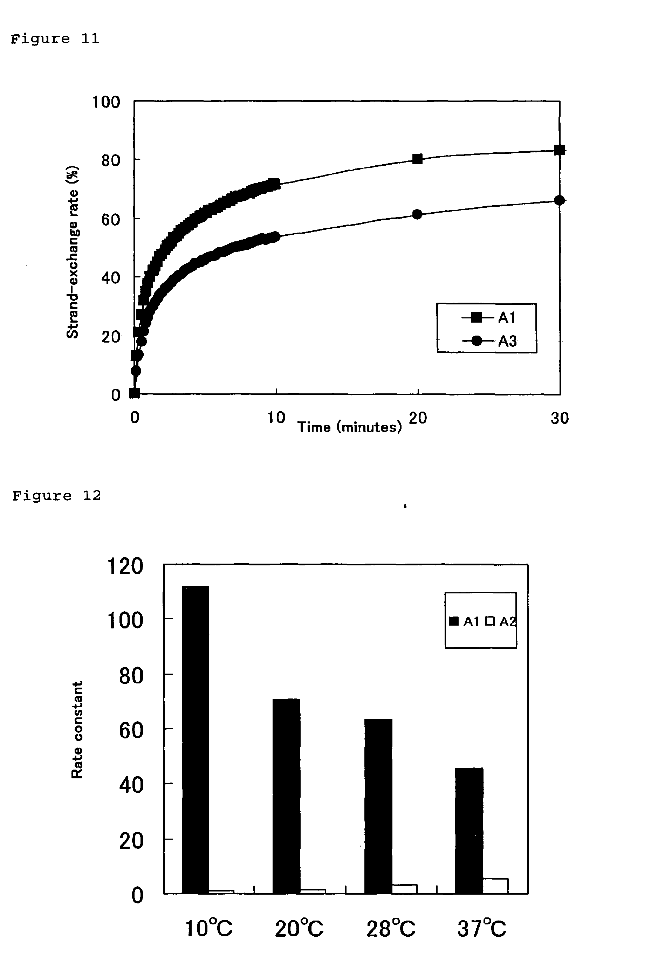

Changes over time in strand-exchange rates after the start of the reaction are shown in Figure 11. Fluorescent strength 5, 10 and 20 minutes after the start of the reaction is also shown in Table 8. The relative strand-exchange rate constants for each single strand are shown in Table 9.

**[Table 8]**

| Sequence | 5 minutes | 10 minutes | 20 minutes |
|---|---|---|---|
| A1 | 155.5 | 172.9 | 188.8 |
| A3 | 103.2 | 114.3 | 124.9 |

**[Table 9]**

| Sequence | Relative strand-exchange rate constant |
|---|---|
| A1 | 1.00 |
| A3 | 0.49 |

As shown in the figure and tables, there was no great difference in strand-exchange rates between the single strand having a mutation corresponding to the double-stranded part of T2 (A3) and the fully complementary single strand (A1) . Judging from these results and the results of Example 4 it appears that the detection sensitivity of a partially double-stranded probe is greatly increased if it is designed so that the mismatched sequence corresponds to the single-stranded part rather than the double-stranded part.

### [Example 5]

Strand-exchange reactions were performed as in Example 4 under 4 sets of conditions with the temperature of the solution set at 10°C, 20 °C, 28°C and 37°C during the strand-exchange reaction.

The relative rate constants for the single strand having the mutation (A2) and the fully complementary single strand (A1) at each temperature are shown in Figure 12. The ratios of the rate for the single chain having the mutation (A2) to the rate for the fully complementary single strand (A1) at each temperature (strand-exchange rate constant for A1 ÷ strand-exchange rate constant for A2) are shown in Table 10.

**[Table 10]**

| Temperature | Rate ratio (A1/A2) |
|---|---|
| 10°C | 112 |
| 20°C | 46 |
| 28°C | 20 |
| 37°C | 9 |

As shown in the figure and table, there was a great difference in strand-exchange rates between the single strand with the mutation and the fully complementary strand at each temperature. The lower the temperature, the greater the difference (rate ratio). From these results it appears that at a temperature range of 10 to 37 °C mismatched sequences can be detected with high sensitivity.

### [Example 6]

Strand-exchange reactions were performed as in Example 4 under 6 sets of conditions with the concentration of the partially double-stranded probe at 12 nM, 9 nM, 6 nM, 4 nM, 3 nM and 1.2 nM and the concentration of the single strand at 60 nM, 45 nM, 30 nM, 20 nM, 15 nM and 6 nM.

The ratios of the rates using the single strand with the mutation (A2) and the full complementary single strand (A1) (strand-exchange rate constant for A1 ÷ strand exchange rate constant for A2) are shown in Table 11.

**[Table 11]**

| Partially double-stranded probe concentration (nM) | Single strand concentration (nM) | Rate ratio (A1/A2) |
|---|---|---|
| 12 nM | 60 nM | 46 |
| 9 nM | 45 nM | 34 |
| 6 nM | 30 nM | 48 |
| 4 nM | 20 nM | 50 |
| 3 nM | 15 nM | 61 |
| 1.2 nM | 6 nM | 50 |

As shown in the table there was a large difference in strand exchange rate between the single strand with the mutation and the fully complementary single strand whatever the combination of concentrations.

### [Example 7]

Strand exchange reactions were performed as in Example 1 except that two kinds of single strands were added, C1 and M1, and the reactions were performed in the presence of the cationic polymer described in International Patent Publication 03/018841, pamphlet (dextran side-chain modified α-poly(L-lysine)). The cationic polymer was added under two sets of conditions, with the N/P ratio (amount of phosphoric acid groups in the ODN relative to amino groups in the cationic polymer) at 0.2 and 0.4.

The relative strand exchange rate constants for each single chain in the presence (N/P = 0.2, 0.4) and absence (N/P = 0) of the cationic surfactant and the ratio of the rate for the single strand with the mutation (M1) to the rate for the fully complementary single strand (C1) (strand exchange rate constant for C1 ÷ strand exchange rate constant for M1) are shown in Table 12.

**[Table 12]**

| N/P | C1 | M1 | Rate ratio (C1/M1) |
|---|---|---|---|
| 0 | 1.00 | 0.02 | 50 |
| 0.2 | 30.35 | 0.36 | 84 |
| 0.4 | 88.66 | 1.25 | 71 |

As shown in the table, the strand exchange rates were much higher when the cationic surfactant was added using either the single strand with the mutation or the fully complementary single strand. From these results, it appears that mismatched sequences can be detected even under conditions that produce a low strand exchange rate (for example, conditions in which the single-stranded probe is only present at low concentrations) if a cationic polymer is also added.

### [Comparative Example 2]

Equal molar amounts of F2 and D2 were added to PBS buffer ([Na⁺] = 150 mM, pH 7.2) . This buffer was heated to 90°C and then gradually cooled to prepare a solution comprising 12 nM of partially double-stranded F2/D2 probe. Keeping this solution at 37°C, A1 and A2 were added as single strands to concentrations of 60 nM to initiate strand-exchange reactions. The progress of strand exchange was detected from the recovery of FITC fluorescence extinguished by DAB.

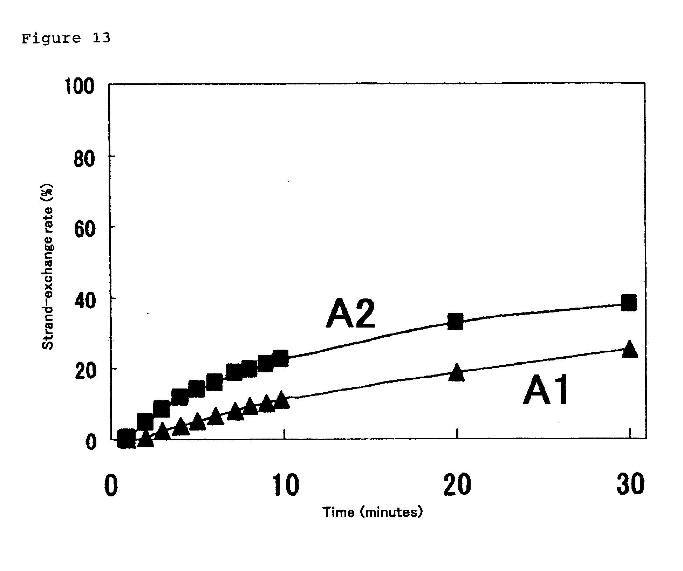

Changes over time in the strand-exchange rates after the start of the reaction are shown in Figure 13. The ratio of the rates of the single strand with the mutation (A1) and the fully complementary single strand (A2) (strand-exchange rate constant of A2 ÷ strand-exchange rate constant of A1) was 2.9.

As shown in the figure, there was no great difference in strand exchange rates between the single strand with the mutation (A1) and the fully complementary single strand. Moreover, the strand-exchange rate was not very high using either single strand. From these results, it appears that conditions such as the GC content, temperature and the like of the single strand to be detected may affect the ability to detect mismatched sequences with high sensitivity.

### [Example 8]

Strand-exchange reactions were performed as in Comparative Example 2 except that tetramethylammonium chloride (TMAC) was added to 3M to the PBS buffer.

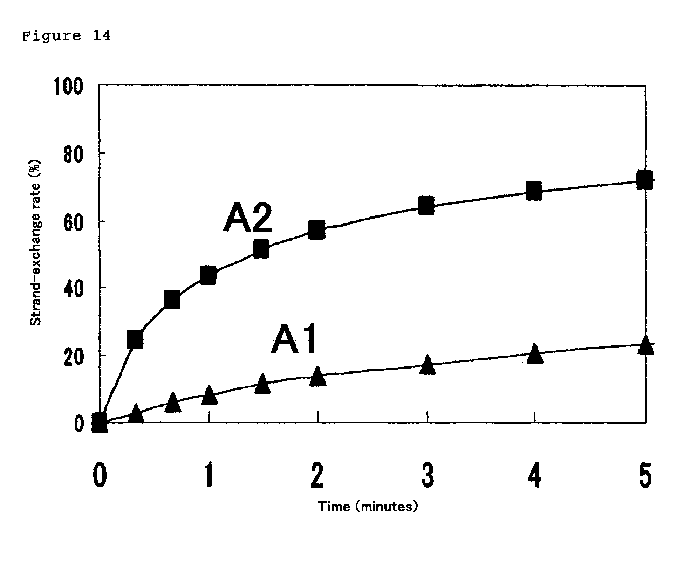

Changes over time in the strand-exchange rates after the start of the reaction are shown in Figure 14. The ratio of the rates for the single strand with the mutation (A1) and the fully complementary single strand (A2) (strand-exchange rate constant of A2 ÷ strand-exchange rate constant of A1) was 10.8.

As shown in the figure, unlike in Comparative Example 2 there was a great difference in strand-exchange rates between the single strand with the mutation (A1) and the fully complementary single strand (A2). The strand-exchange rates were also much higher. From these results, it appears that mismatched sequences can be detected with high sensitivity by adding TMAC even under the conditions of Comparative Example 2.

### [Example 9]

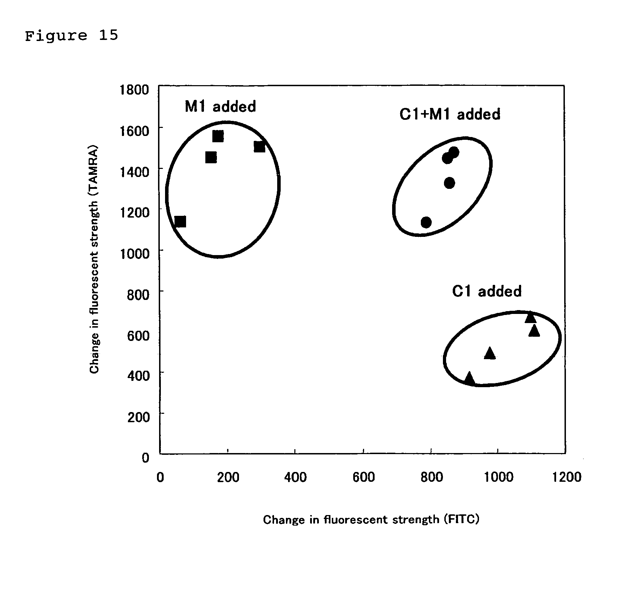

A partially double-stranded F1/D1 probe (FITC labeled) and partially double-stranded T1/D1 probe (TAMRA labeled) were prepared, and each of these two different probes was added to a concentration of 12 nM to PBS buffer ([Na⁺] = 150 mM, pH 7.2) containing 3M of TMAC. M1, C1+M1 or C1 was added to this buffer to a concentration of 60 nM (to a combined concentration of 60 nM of both single strands in the case of C1+M1), and strand-exchange reactions were initiated under room temperature conditions. 5 minutes after the start of the reaction, FITC fluorescent strength and TAMRA fluorescent strength were measured simultaneously with a plate reader. This operation was repeated 4 times for each single strand for a total of 12 times. The 12 measurements are shown in Figure 15.

As shown in the figure, when M1 was added TAMRA fluorescence was detected but little FITC fluorescence was detected, while conversely, when C1 was added FITC fluorescence was detected but almost no TAMRA fluorescence was detected. This indicates that when M1 was added there was strand exchange with the T1/D1 probe but almost none with the F1/D1 probe, while when C1 was added there was almost no strand exchange with the T1/D1 probe but strand exchange occurred with the F1/D1 probe. Since M1 and C1 are fully complementary with T1 and F1, respectively, the results of Figure 15 indicate that both the T1/D1 probe and F1/D1 probe can distinguish their fully complementary single strands with great accuracy.

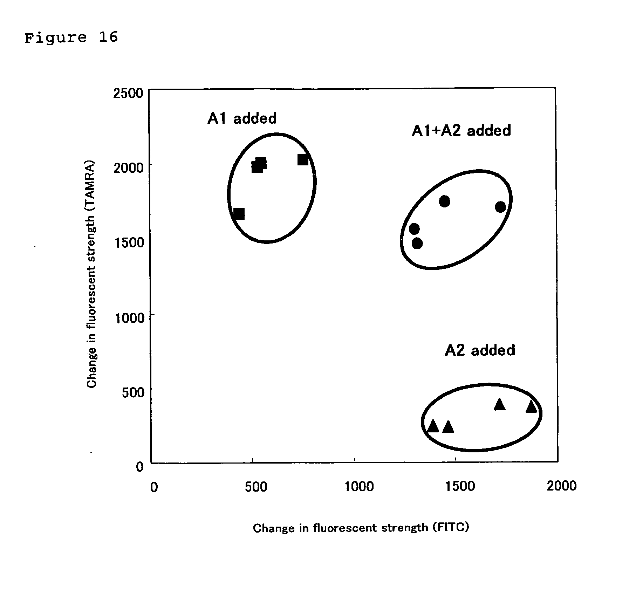

The above experiments with cytochrome P450 gene-related ODNs were also performed using ABC transporter gene-related ODNs. That is, similar experiments were performed using an F2/D2 probe in place of the F1/D1 probe and a T2/D2 probe in place of the T1/D1 probe, and using A2 in place of C1 and A1 in place of M1. The results are shown in Figure 16. As shown in the figure, the partially double-stranded probes recognized the fully complementary single strands very accurately as in the case of the cytochrome P450 gene-related ODNs.

### [Example 10]

Strand-exchange reactions were performed as in Example 4 except that T3/D3 was used as the partially double-stranded probe and 45A1 and 45A2 were added as the single strands.

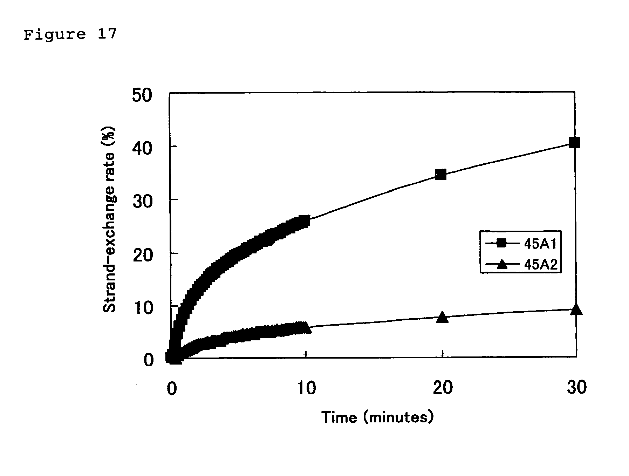

Changes over time in the strand-exchange rates after the start of the reactions are shown in Figure 17. Fluorescent strengths 5, 10 and 20 minutes after the start of each reaction are shown in Table 13. The relative strand-exchange rates for each single strand are shown in Table 14.

**[Table 13]**

| Sequence | 5 minutes | 10 minutes | 20 minutes |
|---|---|---|---|
| 45A1 | 39.0 | 47.5 | 58.9 |
| 45A2 | 15.2 | 17.2 | 19.3 |

**[Table 14]**

| Sequence | Relative strand-exchange rate constant |
|---|---|
| 45A1 | 1.00 |
| 45A2 | 0.11 |

As shown in the figure and tables, there was a great difference in strand-exchange rates between the single strand with the mutation (45A2) and the fully complementary single strand (45A1).

From these results, it appears that mismatched sequences can be detected with great sensitivity even if the double-stranded part is about 40 nucleotides in length.

These specifications also encompass the content described in the specifications and/or drawings of Japanese Patent Application 2003-282311, which forms the basis for the priority of this application. All publications, patents and patent applications cited for this invention can also be referenced as is and included in these specifications.

## Claims

1. A nucleic acid molecule for detecting a nucleic acid molecule, which is a partially double-stranded nucleic acid molecule comprising (a) a single-stranded nucleic acid molecule complementary to the nucleic acid molecule to be detected and (b) one or two single-stranded nucleic acid molecules which hybridize with part of the single-stranded nucleic acid molecule (a), wherein the region of the partially double-stranded nucleic acid molecule which assumes a single-stranded structure is complementary to a region comprising a recognition site in the nucleic acid molecule to be detected.

2. A nucleic acid molecule according to Claim 1, wherein the length of the region that assumes a double-stranded structure is 10 to 200 nucleotides.

3. A nucleic acid molecule according to Claim 1 or 2, wherein the length of the region that assumes a single-stranded structure is 1 to 10 nucleotides.

4. A nucleic acid molecule according to any of Claims 1 through 3, wherein either one of the single-stranded nucleic acid molecule (a) or the single stranded nucleic-acid molecule (b) is labeled with a donor fluorescent dye, while the other is labeled with an acceptor fluorescent dye.

5. A nucleic acid molecule according to any one of Claims 1 through 4, wherein the single-stranded nucleic acid molecule (a) and the single stranded nucleic-acid molecule (b) are connected by means of a linker.

6. A nucleic acid molecule according to Claim 5, wherein the linker is a nucleic acid.

7. A nucleic acid chip comprising a nucleic acid molecule according to any of Claims 1 through 6 fixed on a substrate.

8. A method for detecting a nucleic acid molecule, comprising a step in which a nucleic acid according to any of Claims 1 through 6 is brought into contact with a nucleic acid molecule to be detected under conditions that allow hybridization.

9. A method for detecting a nucleic acid molecule according to Claim 8, wherein a nucleic acid according to any of Claims 1 through 6 is brought into contact with a nucleic acid molecule to be detected in the presence of an amine or quaternary ammonium salt.

10. A method for detecting a nucleic acid molecule according to Claim 8 or 9, wherein a nucleic acid according to any of Claims 1 through 6 is brought into contact with a nucleic acid molecule to be detected in the presence of a cationic polymer.

11. A mismatched sequence detection method for detecting a sequence mismatch between a sample single-stranded nucleic acid molecule and a standard single-stranded nucleic acid molecule, comprising a step in which a nucleic acid molecule which is a partially double-strand nucleic acid molecule comprising (a) a single-stranded nucleic acid molecule complementary to the sample single-stranded nucleic acid molecule and (b) one or two single-stranded nucleic acid molecules which hybridize with part of the single-stranded nucleic acid molecule (a), the region which assumes a single-stranded structure in the partially double-strand nucleic acid molecule being complementary to a region comprising a site where the mismatched sequence is anticipated in the sample single-stranded nucleic acid molecule, is brought into contact with the sample single-stranded nucleic acid under conditions which allow hybridization.

12. A mismatched sequence detection method according to Claim 11, wherein the partially double-strand nucleic acid molecule is brought into contact with the sample nucleic acid molecule in the presence of an amine or quaternary ammonium salt.

13. A mismatched sequence detection method according to Claim 11 or 12, wherein the partially double-strand nucleic acid molecule is brought into contact with the sample nucleic acid molecule in the presence of a cationic polymer.

14. A nucleic acid molecule detection method wherein a first detection probe complementary to a region which does not comprise a recognition site in a nucleic acid molecule to be detected is added to a sample comprising the nucleic acid molecule to be detected, and a second detection probe which comprises a nucleotide sequence identical to that of the first detection probe and which is complementary to the region comprising a recognition site in the nucleic acid molecule to be detected is then added, the nucleic acid molecule to be detected, the first detection probe and the second probe are brought together under conditions that allow hybridization, and nucleic acid molecule detection is then performed using as the marker either binding between the nucleic acid molecule to be detected and the second detection probe, or dissociation between the nucleic acid molecule to be detected and the first detection probe.

15. A nucleic acid molecule detection method according to Claim 14 wherein the nucleic acid molecule to be detected, the first detection probe and the second probe are brought together in the presence of an amine or quaternary ammonium salt.

16. A nucleic acid molecule detection method according to Claim 14 or 15, wherein the nucleic acid molecule to be detected, the first detection probe and the second probe are brought together in the presence of a cationic polymer.
